# EUROPEAN PATENT SPECIFICATION

(11) **EP 4 265 380 B1**
(45) Date of publication and mention of the grant of the patent: **18.11.2026**
(21) Application number: 22169243.7
(22) Date of filing: 21.04.2022
(51) Int. Cl.: B25J 17/00, B25J 19/00, H02K 7/102

(54) **JOINT ARRANGEMENT FOR A ROBOT ARM FOR PROVIDING AN ENERGY-EFFICIENT BRAKING FUNCTION**
GELENKANORDNUNG FÜR EINEN ROBOTERARM ZUR BEREITSTELLUNG EINER ENERGIEEFFIZIENTEN BREMSFUNKTION
AGENCEMENT DE JOINT D'UN BRAS DE ROBOT POUR FOURNIR UNE FONCTION DE FREINAGE À EFFICACITÉ ÉNERGÉTIQUE

(43) Date of publication of application: 25.10.2023
(73) Proprietor: Microsure B.V., 5692 EA Son (NL)
(72) Inventor: van Gerven, Lars, 5382 KC Vinkel (NL); Bosscher, Kasper, 5616 GX Eindhoven (NL); Bezemer, Paul, 5702 TC Helmond (NL)
(74) Representative: K&L Gates LLP

(56) References cited:
- WO-A1-2017/033030
- CN-A- 111 194 517
- CN-U- 215 528 790
- US-A1- 2010 301 785
- US-A1- 2012 235 606
- WANG KAIMENG ET AL: "An Optimized Permanent Magnet Brake Mechanism in Robot Joints", IEEE ACCESS, IEEE, USA, vol. 9, 20 January 2021 (2021-01-20), pages 18278 - 18286, XP011834225, DOI: 10.1109/ACCESS.2021.3053080

## Description

The present invention relates to a joint arrangement for a robot arm having at least one brake device, to a robot arm, and to a method for operating the brake device in the joint arrangement.

The present invention particularly relates to a joint arrangement for a robot arm, especially a robot arm for use in microsurgery, having at least one brake device.

Further, the present invention particularly relates to a robot arm, especially a robot arm for use in microsurgery, comprising said joint arrangement.

Still further, the present invention particularly relates to a method for operating a brake device in a joint arrangement for a robot arm, especially a robot arm for use in microsurgery.

Generally, a brake device such as an electro-magnetic brake can be configured such as to provide its braking effect in a currentless state by use of a magnetic field provided by a permanent magnet. In order to neutralize said braking effect, the magnetic field provided by said permanent magnet is usually displaced by an opposing electro-magnetic field, i.e., in a current-carrying state. However, the power needed for remaining in the state of neutralization of said braking effect generally tends to be at a relatively high level leading to undesired heat generation originating from the electro-magnetic brake. In particular, such an electro-magnetic brake is usually operated by the power being ramped up above a neutralization value for neutralizing the braking effect and by the power being ramped down again below a braking effect value for applying the braking effect again, thereby requiring a lot of power. In this respect, the generated heat, i.e., the high temperatures, can consequently transfer to the environment of the brake, e.g., neighboring elements, which can lead to an uncontrolled change of dimensions of said environment / said elements.

For instance, the above-described effect can be problematic when occurring in applications having the need for high precision such as in surgery procedures, in particular microsurgery or super-microsurgery procedures, such as in joint devices and/or robot devices being used for precise surgical handlings in such procedures.

Further, in view of economic considerations, the above-described effect can also be considered as disadvantageous (e.g., with respect to high power consumption).

WANG KAIMENG ET AL: "An Optimized 1-17 Permanent Magnet Brake Mechanism in Robot Joints", IEEE ACCESS, IEEE, USA, vol. 9, 20 January 2021 (2021-01-20), pages 18278-18286, XP011834225, DOI: 10.1109/ACCESS.2021.3053080 discloses a brake mechanism for robot joints where permanent magnets are used to replace a torque spring, such that large braking torque and low power consumption can be achieved. In conventional brake structures, a torque spring is embedded in the electromagnet. To the contrary, here, the permanent magnet and the electromagnet are distributed on both sides of a brake armature.

Documents US2010/301785A1 and CN111194517A disclose other examples of braking devices.

In view of the above, it is an object of the invention to provide a joint arrangement for a robot arm having at least one brake device, which is capable to be operated in a more economical and more precise manner.

According to the invention, this object is obtained by a joint arrangement for a robot arm, especially a robot arm for use in microsurgery, according to claim 1.

Correspondingly, a joint arrangement for a robot arm, especially a robot arm for use in microsurgery, comprises at least one brake device being configured for engagement and for disengagement and comprising a stator assembly and a rotor assembly, wherein the brake device is further configured to be operable at a minimal power consumption in a disengagement state.

The at least one brake device may be exactly one brake device or more than one brake device.

The brake device may be configured as an electro-magnetic brake.

The minimal power consumption may be a power consumption being necessary for keeping the brake device in a disengagement state, wherein the minimal power consumption may be lower than a power consumption being necessary for switching between an engagement state to said disengagement state.

In particular, the minimal power consumption may differ from said power consumption being necessary for switching between an engagement state to said disengagement state by at least 0 to 5%, preferably 0 to 10% (e.g., 5 to 10%), more preferably 0 to 15% (e.g., 5 to 15%, or 10 to 15%), even more preferably 0 to 20% (e.g., 5 to 20%, or 10 to 20%, or 15 to 20%), or 0 to 25% (e.g., 5 to 25%, or 10 to 25%, or 15 to 25%, or 20 to 25%).

The joint arrangement as described is aiming in one possible arrangement for a 75% power reduction between disengaging and keeping the brake disengaged.

The invention is based on the basic idea that the brake device of the joint arrangement can be operated at a minimal power consumption, to thereby use less power and generate less heat. Accordingly, the joint arrangement can be operated in a more economic manner, wherein, at the same time, it can also be operated in a more accurate manner since thermal expansions are reduced. Operating at the minimal power consumption is possible by making use of a hysteresis effect of the brake device. In particular, the hysteresis effect of the brake device may occur in a range between a power required for disengagement and a power required for engagement. For instance, if the power falls below the power required for disengagement, but stays above the power required for engagement, the brake device can still remain in a stable state of disengagement. Further, since the heat generation can be reduced, the joint arrangement may also be improved with regard to its safety conditions.

The brake device may comprise at least one permanent magnet for providing a braking effect during engagement and being assigned to the rotor assembly, and at least one electromagnet for releasing the braking effect during disengagement and being assigned to the stator assembly, wherein the permanent magnet and the electromagnet may be arranged or arrangeable to each other with a gap between themselves such as to allow the brake device to be operable at a minimal power consumption. In other words, the permanent magnet and the electromagnet may be arranged or arrangeable to each other with a gap between themselves in order to maintain a hysteresis effect between themselves, when operated.

The gap may be smaller than 1 mm ± 10%, in particular smaller than 1 mm ± 5%, particularly smaller than 1 mm ± 3%. For example, the gap may be in a range between 0,2 mm ± 5% and 0,5 mm ± 5%, or the gap may be in a range between 0,5 mm ± 5% and 0,8 mm ± 5%, or the gap may be in a range between 0,8 mm ± 5% and 1 mm ± 5%.

Alternatively, the gap may be smaller than 0,8 mm ± 10%, in particular smaller than 0,8 mm ± 5%, particularly smaller than 0,8 mm ± 3%.

It may also be conceivable that the gap may be smaller than 0,5 mm ± 10%, in particular smaller than 0,5 mm ± 5%, particularly smaller than 0,5 mm ± 3%.

In particular, the gap may be 0,5 mm ± 10%, in particular 0,5 mm ± 5%, particularly 0,5 mm ± 3%.

As one possible example, the gap between brake and brake-disc can be 0,2 mm+0,1 mm and so, it is normally in the range of 0,20 mm to 0,30 mm. The tolerances for the gap can be 0,0171 to 0,329 mm.

The joint arrangement may further comprise at least one motor stack having a rotor assembly and a stator assembly, wherein the brake device may be arranged in connection with or within the motor stack. In particular, the brake device may be arranged in connection with or at a central motor shaft of the motor stack. In other words, the brake device may be arranged for allowing the brake device to apply a braking effect onto the motor stack, in particular, onto the rotor assembly of the motor stack. Hence, the brake device is configured to apply a braking effect onto the motor stack, in particular, onto the rotor assembly of the motor stack.

The rotor assembly of the brake device may be configured as a part of the rotor assembly of the motor stack, and the stator assembly of the brake device may be configured as a part of the stator assembly of the motor stack. For example, the rotor assembly of the brake device may be fixed to the rotor assembly of the motor stack by a shaft hub connection, thereby forming a part of the rotor assembly of the motor stack. However, any other suitable connection may also be applicable. Hence, the braking effect applicable by the brake device may be provided to the motor stack in a more direct manner, e.g., without any intermediate element (e.g., transmitting elements).

The motor stack may comprise at least one spring, in particular at least one leaf spring, being configured to provide a spring force to the brake device. For example, the spring may be arranged in connection with or at a/the central motor shaft of the motor stack, to thereby provide said spring force to the brake device also being arranged in connection with or at said central motor shaft. In other words, the spring force may be provided to the brake device via the central motor shaft. In particular, the spring may be configured to provide a/the spring force to the brake device for intensifying a/the hysteresis effect of the brake device.

Further, according to the invention, the above-described object is also obtained by a robot arm, especially a robot arm for use in microsurgery, according to claim 7.

Correspondingly, a robot arm, especially a robot arm for use in microsurgery, comprises a joint arrangement, for example, a/the joint arrangement as described herein, e.g., as described above and/or further below.

The robot arm may further comprise one or more further joint arrangements being particularly configured as described herein, e.g., as described above and/or further below.

In other words, the robot arm may comprise one or more joint arrangements, wherein at least one joint arrangement may be configured as described herein, e.g., as described above and/or further below.

Still further, according to the invention, the above-described object is also obtained by a method for operating a brake device in a joint arrangement for a robot arm, especially a robot arm for use in microsurgery, according to claim 8.

Correspondingly, a method for operating a brake device in a joint arrangement (e.g., a/the joint arrangement as described herein, e.g., as described above and/or further below) for a robot arm (e.g., a/the robot arm as described herein, e.g., as described above and/or further below), especially a robot arm for use in microsurgery, the method at least comprising the following step: the brake device is provided and configured for engagement and for disengagement, the brake device comprising a stator assembly and a rotor assembly, wherein the brake device is further operated at a minimal power consumption in a disengagement state.

Accordingly, as described above, due to the method, the brake device of the joint arrangement can be operated at a minimal power consumption, to thereby use less power and generate less heat. Hence, the joint arrangement can be operated in a more economic manner, wherein, at the same time, it can also be operated in a more accurate manner since thermal expansions are reduced.

The brake device may further be configured for engagement at an engagement threshold value and for disengagement at a disengagement threshold value being higher than the engagement threshold value. Hence, the brake device may reliably function as a holding brake. In case of a loss of power, the joint arrangement may be prevented to continue moving by the brake device, since the engagement threshold value may be smaller than the disengagement threshold value.

The method may further comprise the following steps: providing full power to the brake device by applying Pulse Width Modulation (PWM) for disengaging the brake device; reducing the power provided to the brake device to a first predetermined power value by applying PWM, wherein the first predetermined power value is set between the disengagement threshold value and the engagement threshold value; and maintaining the power provided to the brake device at the first predetermined power value by applying PWM for keeping the brake device in disengagement. Hence, the brake device may be switched to its disengagement state by the application of a full power PWM signal and, subsequently, may be maintained therein due to the application of the reduced power PWM signal being lower than the disengagement threshold value and higher than the engagement threshold value, thereby making use of the hysteresis of the brake device and, accordingly, saving power and generating less heat.

The method may further comprise reducing the power provided to the brake device to a second predetermined power value by applying PWM for engaging the brake device, wherein the second predetermined power value may be set below the engagement threshold value.

The first predetermined power value may be half of the full power being providable or provided to the brake device by applying pulse width modulation (PWM) for disengaging the brake device. Hence, due to halving the power, an energy reduction of approximately 25% may be achieved or achievable.

Additionally and/or alternatively, by halving the voltage a power reduction of 75% can be achieved.

The power reduction can also be influenced by PWM, where the PWM equivalent of halving the voltage is applied giving a power reduction of approx. 75%.

The second predetermined power value may be zero, i.e., substantially no power.

The full power being providable or provided to the brake device by applying PWM for disengaging the brake device may include a 24V signal. In this respect, the first predetermined power value may be 12V. However, any other signal may also be conceivable with respect to the full power, such as a 12V signal or a 48V signal. The second predetermined power value may be 0V (i.e., zero).

The disengagement threshold value may be 5/8 of the full power. For example, in case of the full power including a 24V signal, the disengagement threshold value may be 15V.

The engagement threshold value may be 1/3 of the full power. For example, in case of the full power including a 24V signal, the engagement threshold value may be 8V.

The step of providing full power to the brake device by applying PWM for disengaging the brake device may be maintained for a first predetermined time period, and the step of maintaining the power provided to the brake device at the first predetermined power value by applying PWM for keeping the brake device in disengagement may be maintained for a second predetermined time period, wherein the second predetermined time period may be longer than the first predetermined time period. Hence, full power may (e.g., only) be used for switching the brake device into the disengagement state for a relatively short period of time (e.g., compared to maintaining the disengagement state), thereby ensuring that the brake device may be operated in an economic way as well as that excessive heat generation may be prevented.

The brake device may comprise at least one permanent magnet for providing a braking effect during engagement and at least one electromagnet for releasing the braking effect during disengagement. The electromagnet may be configured to provide an electro-magnetic field for opposing the magnetic field of the permanent magnet for neutralization of the braking effect of the brake device.

The brake device may be arranged in connection with or within a motor stack of the joint arrangement.

The motor stack may comprise at least one spring, in particular at least one leaf spring, being configured to provide a spring force to the brake device.

It should be understood that any feature described in connection with the joint arrangement and/or with the robot arm may also be part of the method for operating a brake device in a joint arrangement, and vice versa. It should be further understood that any advantage and/or property described in connection with the joint arrangement and/or with the robot arm may also apply to the method for operating a brake device in a joint arrangement, and vice versa.

Further details and advantages of the present invention shall now be disclosed in connection with the drawings, where:
- **Fig. 1**: schematically shows a cross sectional view of a joint arrangement for a robot arm according to an exemplary embodiment of the invention;
- **Fig. 2A**: schematically shows a cross sectional view of a motor stack of a joint arrangement for a robot arm according to another exemplary embodiment of the invention;
- **Fig. 2B**: schematically shows a cross sectional view of a part of the motor stack of Fig. 2A;
- **Fig. 3**: schematically shows a robot arm according to another exemplary embodiment of the invention;
- **Fig. 4**: shows a diagram depicting a method for operating a brake device in a joint arrangement for a robot arm according to another exemplary embodiment of the invention;
- **Fig. 5**: schematically shows a diagram depicting an operating behavior of the method of Fig. 4;
- **Fig. 6**: shows a diagram depicting an assembly method of the motor stack of Fig. 2A according to another exemplary embodiment of the invention;
- **Fig. 7**: schematically shows a part of the assembly method of Fig. 6;
- **Fig. 8**: schematically shows a part of the assembly method of Fig. 6;
- **Fig. 9**: schematically shows a part of the assembly method of Fig. 6;
- **Fig. 10**: schematically shows a part of the assembly method of Fig. 6;
- **Fig. 11**: schematically shows a part of the assembly method of Fig. 6;
- **Fig. 12A-12C**: schematically show alternatives of a part of the assembly method of Fig. 6, respectively;
- **Fig. 13**: schematically shows a part of the assembly method of Fig. 6; and
- **Fig. 14**: schematically shows a part of the assembly method of Fig. 6.

Identical or functionally equivalent elements are denoted with the same reference signs throughout the figures.

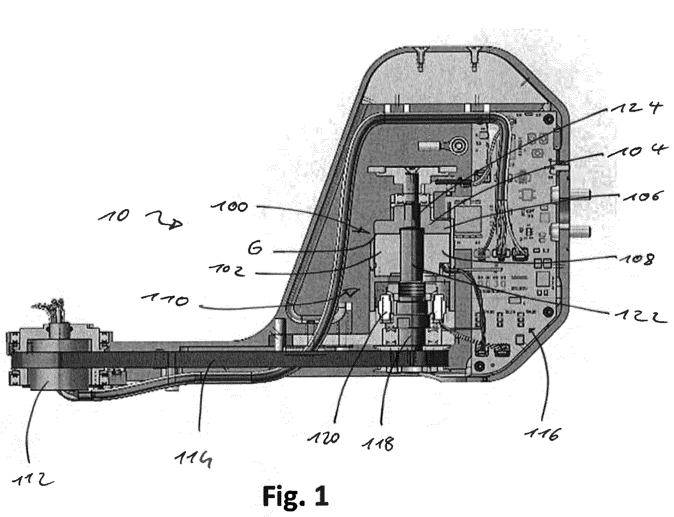

Turning to **Fig. 1****,** a cross sectional view of a joint arrangement 10 for a robot arm 20 according to an exemplary embodiment of the invention is schematically shown.

The joint arrangement 10 comprises a brake device 100.

The brake device 100 is configured as an electro-magnetic brake.

The brake device 100 is configured for engagement and for disengagement.

Engagement corresponds to a state, in which a braking effect is applied by the brake device 100, and disengagement corresponds to a state, in which the breaking effect is not applied by the brake device 100.

The brake device 100 is further configured to be operable at a minimal power consumption in a/the disengagement state, which will be described further below.

The brake device 100 comprises a stator assembly 102 and a rotor assembly 104.

The brake device 100 further comprises a permanent magnet 106 for providing a braking effect during engagement.

The permanent magnet 106 is assigned to the rotor assembly 104.

The permanent magnet 106 is constructed to be substantially axially symmetric relative to a central axis.

The brake device 100 also comprises an electromagnet 108 for releasing the braking effect during disengagement.

The electromagnet 108 is assigned to the stator assembly 102.

The electromagnet 108 is constructed to be substantially axially symmetric relative to a central axis.

The permanent magnet 106 and the electromagnet 108 are disposed opposite to each other in such a way that their symmetric axes coincide. In this respect, the permanent magnet 106 and the electromagnet 108 are arranged or arrangeable to each other with a gap G between themselves.

The gap G between the permanent magnet 106 and the electromagnet 108 is dimensioned such as to allow the brake device 100 to be operable at a minimal power consumption, e.g., to be allowed to make use of a hysteresis effect between themselves, when operated, in order to be operable at a minimal power consumption.

In the present embodiment, the gap G is 0,5 mm ± 10%.

However, it may also be conceivable that the gap G is alternatively dimensioned such as the following: the gap may be smaller than 1 mm ± 10%, in particular smaller than 1 mm ± 5%, particularly smaller than 1 mm ± 3%. For example, the gap may be in a range between 0,2 mm ± 5% and 0,5 mm ± 5%, or the gap may be in a range between 0,5 mm ± 5% and 0,8 mm ± 5%, or the gap may be in a range between 0,8 mm ± 5% and 1 mm ± 5%. Alternatively, the gap may be smaller than 0,8 mm ± 10%, in particular smaller than 0,8 mm ± 5%, particularly smaller than 0,8 mm ± 3%. It may also be conceivable that the gap may be smaller than 0,5 mm ± 10%, in particular smaller than 0,5 mm ± 5%, particularly smaller than 0,5 mm ± 3%.

As can be seen in Fig. 1, the joint arrangement 10 further comprises a motor stack 110.

The motor stack 110 is disposed in the joint arrangement 10 for driving a pulley 112 via a belt 114 for transmitting a torque.

Further, in the exemplary embodiment, the joint arrangement 10 also comprises a control unit 116 for controlling the motor stack 110 and/or the brake device 100.

However, it may also be conceivable that the control unit 116 may be configured as an element being separate from the joint arrangement 10 and having a connection with the joint arrangement 10 for signal transmission.

The motor stack 110 comprises a rotor assembly 118 and a stator assembly 120.

The rotor assembly 118 of the motor stack 110 is partly formed by a central motor shaft 122 of the motor stack 10.

The brake device 100 is arranged in connection with or within the motor stack 110.

In particular, the brake device 100 is arranged at said central motor shaft 122 of the motor stack 110.

Hence, the brake device 100 is arranged for allowing the brake device 100 to apply a braking effect onto the motor stack 110, in particular, onto the rotor assembly 118 of the motor stack 100.

In this respect, the rotor assembly 104 of the brake device 100 is rotatable together with the rotor assembly 118 of the motor stack 110, and the stator assembly 102 of the brake device 100 is non-rotatable, e.g., fixed, together with the stator assembly 120 of the motor stack 110.

Therefore, the rotor assembly 104 of the brake device 100 can be seen as being configured as a part of the rotor assembly 118 of the motor stack 110, and the stator assembly 102 of the brake device 100 can be seen as being configured as a part of the stator assembly 120 of the motor stack 110.

For example, in the present embodiment, the rotor assembly 104 of the brake device 100 is fixed to the rotor assembly 118 of the motor stack 110 by a shaft hub connection 124, thereby forming a/the part of the rotor assembly 118 of the motor stack 110.

Accordingly, the rotor assembly 118 of the motor stack 110 and the rotor assembly 104 of the brake device 100 are stopped or stoppable together in case of engagement of the brake device 100 and are released or releasable again together in case of disengagement of the brake device 100.

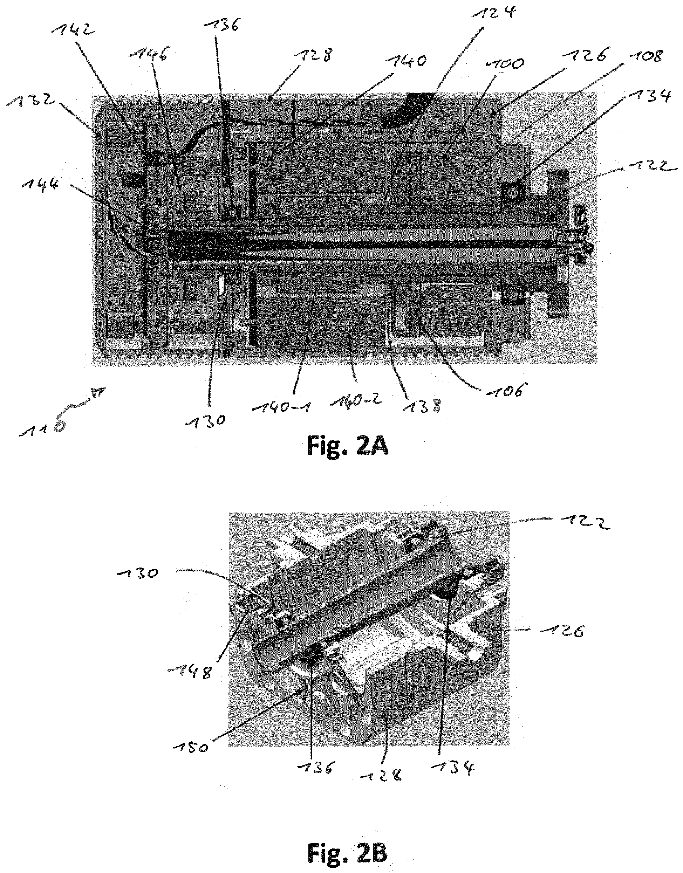

Now turning to **Figs. 2A and 2B****,** a cross sectional view of a motor stack 110 of a joint arrangement 10 for a robot arm 20 according to another exemplary embodiment of the invention is schematically shown in more detail.

The motor stack 110 shown in Figs. 2A and 2B is substantially the same as described above, in particular, with respect to the joint arrangement 10 shown in Fig. 1, so that only the differences shall be described in the following.

It should be understood that the motor stack 110 of Fig. 1 may be substituted with the motor stack 110 of Figs. 2A and 2B. Hence, the joint arrangement 10 of Fig. 1 may alternatively comprise the motor stack 110 of Fig. 2A and 2B.

In other words, the motor stack 110 of Figs. 2A and 2B may be configured for integration into a joint arrangement 10 for a robot arm 20, in particular, in the joint arrangement 10 of Fig. 1.

Referring to Fig. 2A, the motor stack 110 further comprises a first housing 126, a second housing 128, an intermediate flange 130 and a cover 132.

The first housing 126 is connected or connectable to the second housing 128, and the second housing 128 is connected or connectable to the intermediate flange 132 and the cover 132.

A respective sealing is disposed between the first housing 126 and the second housing 128, and between the second housing 128 and the cover 132.

The motor shaft 122 is substantially enclosed by the cover 130 and the first and the second housing 126, 128 and protrudes through the first housing 126 for being able to output a torque.

The motor stack 110 further comprises a first bearing 134 and a second bearing 136.

The first and second bearings 134, 136 are configured as ball bearings, respectively. However, any other bearing configuration may also be conceivable.

The first bearing 134 is disposed in the first housing 126 for rotatably holding the motor shaft 122 at a first position.

In particular, the first bearing 134 is disposed in an/the opening of the first housing 126, through which the motor shaft 122 extends for protruding from the first housing 126 in order to output a/the torque.

The brake device 100 is arranged in the first housing 126 and adjacent to the first bearing 134.

In particular, the electromagnet 108 of the brake device 100 is fixed to the first housing 126.

The motor shaft 122 extends through a central passage formed in the electromagnet 108 such as to be able to rotate.

The permanent magnet 106 is fixed to a flange 138 of the motor stack 110, i.e., the motor stack 110 further comprises a flange 138 for connecting to the brake device 100, in particular, to the permanent magnet 106. However, it may also be conceivable that the flange 138 may be configured as a part of the brake device 100.

Said flange 138 (together with the permanent magnet 106) is fixed to the motor shaft 122 by the before-mentioned shaft hub connection 124.

In this respect, the permanent magnet 106 (on the flange 138) and the electromagnet 108 are arranged to form the above-described gap G between themselves.

The second bearing 136 is disposed in the intermediate flange 130 being fixed to the second housing 128 for rotatably holding the motor shaft 122 at a second position.

Hence, the second housing 128 is fixed to the first housing 126 at one end thereof and to the cover 132 at the opposite end thereof, wherein the intermediate flange 130 and, accordingly, the second bearing 136 are arranged on said opposite end between the cover 132 and the second housing 128.

As can be seen in Fig. 2B, the intermediate flange 130 is pretensioned against the second housing 128 by at least one pretension spring 148, for example, three pretension springs 148 being equally arrangeable at the intermediate flange 130 with respect to (e.g., around) a central axis or, in a mounted state, the motor shaft 122.

The pretension spring 148 is configured as a compression spring.

Since the intermediate flange 130 is pretensioned by the pretension spring 148, the second bearing 136 is also pretensioned, thereby eliminating axial backlash.

Additionally, the motor stack 110 comprises at least one further spring 150, in particular at least one leaf spring 150.

The leaf spring 150 is fixed to the second housing 128 on one side thereof and in communication with the motor shaft 122 and/or the intermediate flange 130 and/or the rotatable part of the second bearing 136 on the other side thereof.

The leaf spring 150 is configured to provide a spring force to the brake device 100, in particular to the permanent magnet 106 of the brake device 100 via the motor shaft 122. Hence, due to said spring force provided by the leaf spring 150, a/the hysteresis effect of the brake device 100 can be intensified.

Further, the leaf spring 150 can support the second bearing in a radial direction (via the motor shaft 122).

A driving assembly 140 is arranged in the first and the second housing 126, 128 and adjacent to the second bearing 136.

In other words, the motor stack 110 comprises a driving assembly 140 for driving, e.g., for generating torque.

The driving assembly 140 (e.g., a motor rotor 140-1, a motor stator 140-2, and a motor PCB) is partly arranged at the motor shaft 122 in a rotatable manner (i.e., rotatable part 140-1 of the driving assembly 140) and at the first and/or the second housing 126, 128 in a non-rotatable manner (i.e., non-rotatable part 140-2 of the driving assembly 140) as generally known in the art.

The driving assembly 140 is connected to a drive PCB 142, which is arranged in the cover 132 and held therein by a PCB holding structure 144.

The drive PCB 142 is signally connected to an encoder 146 disposed at an end of the motor shaft 122.

Hence, the motor stack 110 also comprises said drive PCB 142 for controlling the driving assembly 140 as generally known in the art, said PCB holding structure 144, and said encoder 146 for determining the angular position of the motor shaft 122 as generally known in the art.

Accordingly, with reference to Fig. 2A, when seen along the motor shaft 122, the above-described elements are substantially disposed in an order of the first bearing 134, the electromagnet 108, the permanent magnet 106, the flange 138, the rotatable part 140-1 of the driving assembly 140, the second bearing 136, and the encoder 146.

It should be understood that additional elements may be disposed on or in connection with the motor shaft 122 such as one or more bushings, one or more retaining rings, one or more sleeves, one or more spacers, one or more retaining washers and/or one or more slotted / grooved nuts (e.g., as exemplarily shown in Fig. 2A).

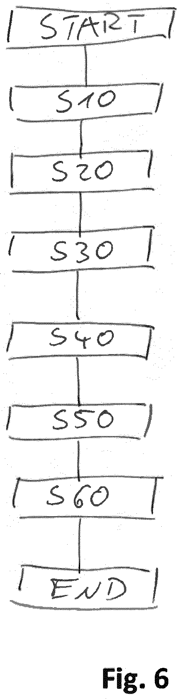

Examples of such additional elements are exemplarily shown and exemplarily described in connection with a/the assembly method of Figs. 6 to 14 further below.

With reference to Figs. 2A and 2B, the brake device 100 can provide a/the braking effect to the motor shaft 122 by the permanent magnet 106 (during engagement) and can release the braking effect therefrom by the electromagnet 108 (during disengagement).

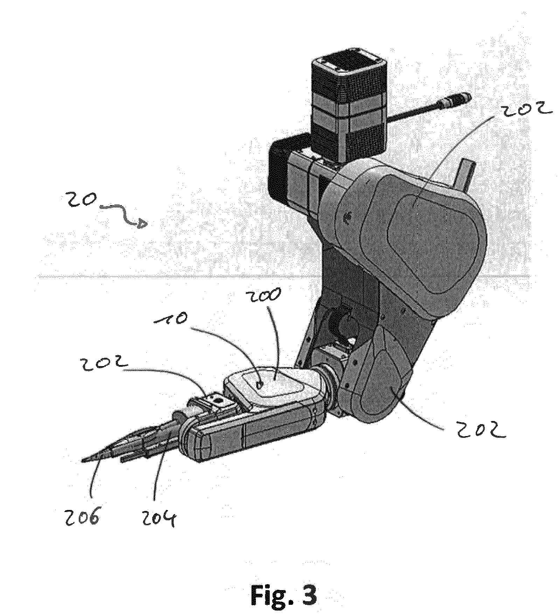

Now turning to Fig. 3, a robot arm 20 according to another exemplary embodiment of the invention is schematically shown.

The robot arm 20 is particularly configured for use in microsurgery.

The robot arm 20 comprises a joint arrangement 10, for example, a/the joint arrangement 10 as described herein, e.g., as described above and/or further below.

The joint arrangement 10 forms a sub arm 200 of the robot arm 10.

The robot arm 10 further comprises further sub arms 202 for articulation purposes.

The respective sub arms 202 may each comprise at least one brake device 100 and/or at least one motor stack 110 as described herein. Hence, the sub arms 202 may also be formed by one or more further joint arrangements being particularly configured as described herein, e.g., as described above and/or further below.

In other words, the robot arm 20 may comprises one or more joint arrangements 10, wherein at least one joint arrangement 10 may be configured as described herein, e.g., as described above and/or further below.

As can be seen in Fig. 3, the robot arm 20 additionally comprises an adapter assembly 204 for holding a surgical instrument 206, to thereby be usable in surgery, in particular microsurgery.

Said adapter assembly 204 is rotatably connected to the joint arrangement 10.

Said adapter assembly 204 is rotatable by applying a torque provided or providable by the motor stack 110 in the joint arrangement 10, wherein the torque is transferred or transferable to the adapter assembly 204 via the belt 114 and the pulley 112 in the joint arrangement 10.

Further, the rotation of the adapter assembly 205 can be stopped by operating the brake device 100 assigned to said motor stack 110.

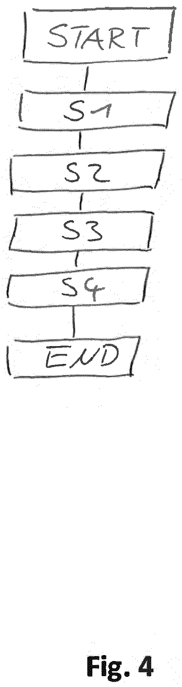

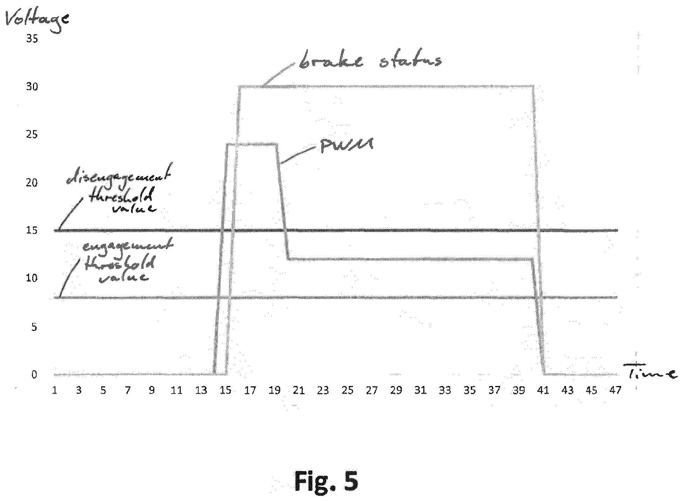

Turning to **Figs. 4** **and** **5****,** a diagram depicting a method for operating a brake device 100 in a joint arrangement 10 (e.g., the joint arrangement 10 as described in connection with Figs. 1 to 2B) for a robot arm 20 (e.g., the robot arm 20 as described in connection with Fig. 3) according to another exemplary embodiment of the invention is shown in Fig. 4, and a diagram depicting an operating behavior of the method of Fig. 4 is schematically shown in Fig. 5.

The method for operating a/the brake device 100 in a/the joint arrangement 10 for a/the robot arm at least comprises the following step: the brake device 100 is provided, wherein the brake device 100 is configured for engagement and for disengagement and comprises a stator assembly 102 and a rotor assembly 104, wherein the brake device 100 is further operated at a minimal power consumption in a disengagement state.

Accordingly, due to the method, the brake device 100 of the joint arrangement 10 can be operated at a minimal power consumption, to thereby use less power and generate less heat. Hence, the joint arrangement 10 can be operated in a more economic manner, wherein, at the same time, it can also be operated in a more accurate manner since thermal expansions are reduced.

The brake device 100 is configured to engage at an engagement threshold value.

Further, the brake device 100 is configured to disengage at a disengagement threshold value.

The disengagement threshold value is higher than the engagement threshold value.

The disengagement threshold value and the engagement threshold value respectively are smaller than a full power provided or providable for operating the brake device 100 (e.g., as intended and/or without misusage).

Hence, the brake device 100 can reliably function as a holding brake. In case of a loss of power, the joint arrangement 10 can be prevented to continue moving by the brake device 100, since the engagement threshold value is smaller than the disengagement threshold value.

In particular, with reference to Fig. 4, the method comprises the step of providing (S1) full power to the brake device 100 by applying Pulse Width Modulation (PWM) for disengaging the brake device 100.

In the present embodiment, said full power being providable or provided to the brake device 100 includes a 24V signal (see PWM signal in Fig. 5). However, any other signal may also be conceivable with respect to the full power, such as a 12V signal or a 48V signal.

The disengagement threshold value is 5/8 of the full power.

Hence, the full power including a 24V signal in the present embodiment, the disengagement threshold value is 15V (see disengagement threshold value in Fig. 5).

The engagement threshold value is 1/3 of the full power.

Hence, the full power including a 24V signal in the present embodiment, the engagement threshold value is 8V (see engagement threshold value in Fig. 5).

After the disengagement of the brake device 100 due to providing full power thereto, e.g., after the brake device 100 is switched to its (e.g., stable) disengagement state by providing full power thereto, the power is reduced to a first predetermined power value by applying PWM, step S2.

As can be seen in Fig. 5, the first predetermined power value is set between the disengagement threshold value and the engagement threshold value.

In other words, the step of reducing (S2) the power provided to the brake device 100 to a/the first predetermined power value by applying PWM is applied, wherein the first predetermined power value is set between the disengagement threshold value and the engagement threshold value.

The first predetermined power value is half of the full power being providable or provided to the brake device 100 by applying PWM for disengaging the brake device 100. In this respect, the first predetermined power value is 12V in the present embodiment (see PWM signal in Fig. 5).

Hence, due to halving the power, an energy reduction of approximately 25% may be achieved or achievable in the present embodiment.

Then, the power is maintained at the first predetermined power value by applying PWM for keeping the brake device 100 in disengagement, i.e., its (stable) disengagement state, step S3.

In other words, the step of maintaining (S3) the power provided to the brake device 100 at the first predetermined power value by applying PWM is applied for keeping the brake device 100 in disengagement.

Hence, the brake device 100 is switched or switchable to its disengagement state by the application of a/the full power PWM signal and, subsequently, is maintained or maintainable therein due to the application of the reduced power PWM signal being lower than the disengagement threshold value and higher than the engagement threshold value, thereby making use of the hysteresis of the brake device 100 and, accordingly, saving power and generating less heat.

Since the brake device 100 can be kept in disengagement, i.e., its (stable) disengagement state, by applying the PWM signal set below the disengagement threshold value due to exploitation of the hysteresis effect, the brake device 100 can be operated at said minimal power consumption in the disengagement state.

Accordingly, the brake device 100 of the joint arrangement 10 can be operated using less power and, therefore, generate less heat. Accordingly, the joint arrangement 10 can be operated in a more economic manner, wherein, at the same time, it can also be operated in a more accurate manner since thermal expansions are reduced. Further, since the heat generation can be reduced, the joint arrangement 10 can also be improved with regard to its safety conditions.

The above-described step of providing (S1) full power to the brake device 100 by applying PWM for disengaging the brake device 100 is maintained for a first predetermined time period.

Further, the above-described step of maintaining (S3) the power provided to the brake device 100 at the first predetermined power value by applying PWM for keeping the brake device 100 in disengagement is maintained for a second predetermined time period.

As can be seen in Fig. 5 as being schematically depicted, the second predetermined time period is (e.g., much) longer than the first predetermined time period.

Hence, full power is (e.g., only) used for switching the brake device 100 into the disengagement state for a relatively short period of time (e.g., compared to maintaining the disengagement state), thereby ensuring that the brake device 100 is operated in an economic way as well as that excessive heat generation is prevented.

In case of the brake device 100 to be switched back to engagement, i.e., its engagement state, the power is reduced again to a second predetermined power value by applying PWM, step S4.

The second predetermined power value is set below the engagement threshold value, i.e., below 8V in the present embodiment. As can be seen in Fig. 5, the second predetermined power value is 0V in the present embodiment (see PWM signal in Fig. 5).

In other words, the method further comprises the step of reducing (S4) the power provided to the brake device 100 to a/the second predetermined power value by applying PWM for engaging the brake device 100, wherein the second predetermined power value is set below the engagement threshold value.

It should be understood that any feature described in connection with the joint arrangement 10 and/or with the robot arm 20 may also be part of the method for operating a brake device 100 in a joint arrangement 10, and vice versa. It should be further understood that any advantage and/or property described in connection with the joint arrangement 10 and/or with the robot arm 20 may also apply to the method for operating a brake device 100 in a joint arrangement 10, and vice versa.

In this respect, for example, the brake device 100 comprises at least one permanent magnet 106 for providing a braking effect during engagement and at least one electromagnet 108 for releasing the braking effect during disengagement.

Accordingly, in another example, the electromagnet 108 is configured to provide an electro-magnetic field for opposing the magnetic field of the permanent magnet 106 for neutralization of the braking effect of the brake device 100.

Accordingly, in another example, the brake device 100 is arranged in connection with or within a motor stack 110 of the joint arrangement 10.

Accordingly, in a still further example, the motor stack 110 comprises at least one spring 150, in particular at least one leaf spring 150, being configured to provide a spring force to the brake device 100.

Turning to **Fig. 6****,** the present invention additionally provides an assembly method of a/the motor stack 110 as described herein in order to help understanding the invention, in particular, the claimed invention, holistically. Hence, Fig. 6 shows a diagram depicting the assembly method of the motor stack 110 of Fig. 2A according to another exemplary embodiment of the invention.

It should be understood that any feature described in connection with the joint arrangement 10 and/or with the robot arm 20 may also be part of said assembly method, and vice versa. It should be further understood that any advantage and/or property described in connection with the joint arrangement 10 and/or with the robot arm 20 may also apply to said assembly method, and vice versa.

In a, e.g., a first, step S10, the flange 138 is assembled with the permanent magnet 106.

In other words, the assembly method comprises the step of assembling (S10) a flange sub assembly.

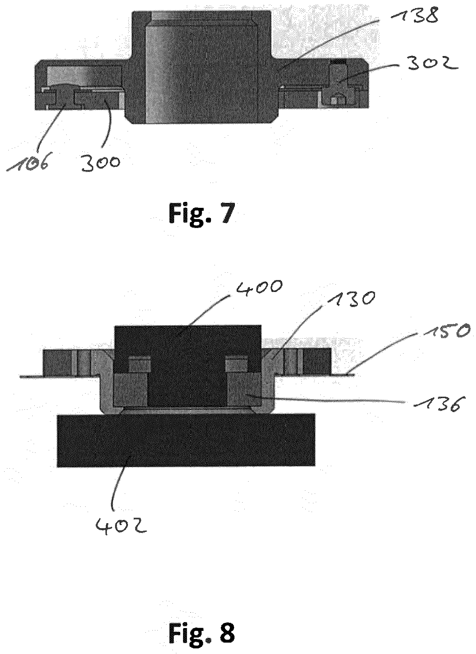

The flange sub assembly is substantially formed by the flange 138 and the permanent magnet 106 (see Fig. 7).

In a further, e.g., a second, step S20, the intermediate flange 130 is assembled with the second bearing 136 and the leaf spring 150.

In other words, the assembly method further comprises the step of assembling (S20) an intermediate flange sub assembly.

The intermediate flange sub assembly is substantially formed by the intermediate flange 130, the second bearing 136 and the leaf spring 150 (see Fig. 8).

In a further, e.g., a third, step S30, the first housing 126 is assembled with a plurality of elements of the motor stack 110 as described with respect to Fig. 2A, such as the flange 138 and the permanent magnet 106, i.e., the before-mentioned flange sub assembly, thereby substantially forming a first housing sub assembly.

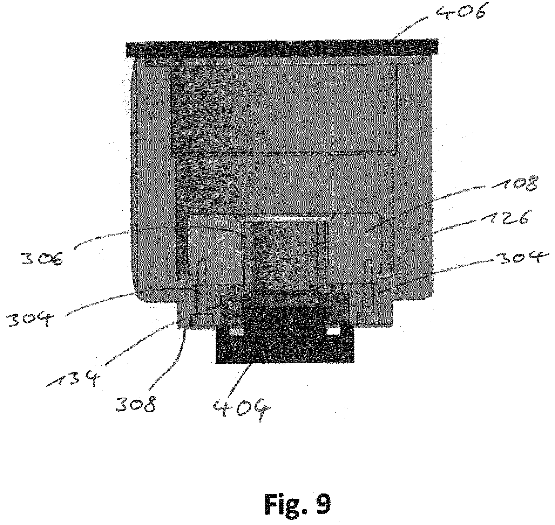

In other words, the assembly method further comprises the step of assembling (S30) a/the first housing sub assembly (see Figs. 9 to 12C).

In a further, e.g., a fourth, step S40, the second housing 128 is mounted to the first housing 126, i.e., to the first housing sub assembly.

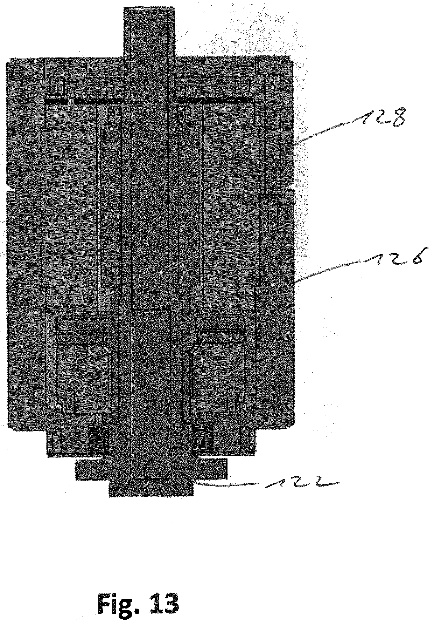

In other words, the assembly method further comprises the step of mounting (S40) the second housing 128 to a/the first housing sub assembly (see Fig. 13).

In a further, e.g., a fifth, step S50, the intermediate flange sub assembly is mounted to the second housing 128 and to the first housing sub assembly having the second housing 128 mounted thereto.

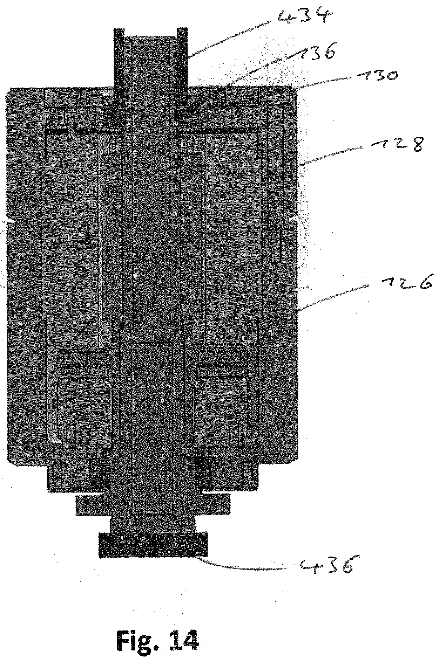

In other words, the assembly method further comprises the step of mounting (S50) the intermediate flange sub assembly to the second housing 128 and to the first housing sub assembly (see Fig. 14).

In a further, e.g., a sixth, step S60, the cover 132 is assembled with a plurality of elements of the motor stack 110 as described with respect to Fig. 2A, thereby substantially forming a cover sub assembly, and then the cover sub assembly is mounted to the assembly resulting from the (e.g., fifth) step S50, i.e., substantially formed by the intermediate flange sub assembly, the second housing 128 and the first housing sub assembly (not particularly shown in the figures).

Accordingly, a/the motor stack 110, e.g., the motor stack 110 of Fig. 2A, can be assembled by performing steps S10 to S60.

It should be understood that the above-described order of steps may be changed with respect to technical reasonability. For example, the first step S10 and the second step S20 may be interchanged and/or may be applied during the third step S30 and/or the second step S20 may be applied after the fourth step S40, and so on. It should be further understood that any of the above-described steps may also be applied in a parallel manner with respect to technical reasonability.

Turning to Fig. 7, a part of the assembly method of Fig. 6 is schematically shown.

In particular, the flange sub assembly of step S10 is schematically shown.

The flange sub assembly comprises the flange 138, a brake armature 300 of the brake device 100 having the permanent magnet 106 (e.g., at least one permanent magnet 106) arranged therein or thereto, and at least one fastener 302.

The fastener 302 is arranged to fix the brake armature 300 having the permanent magnet 106 to the flange 138.

In other words, the permanent magnet 106 is fixed to the flange 138 by means of at least one fastener 302.

The fastener 302 is configured as a screw fastener.

Turning to Fig. 8, a part of the assembly method of Fig. 6 is schematically shown.

In particular, the intermediate flange sub assembly of step S20 is schematically shown.

The intermediate flange sub assembly comprises the intermediate flange 130, the second bearing 136 and the leaf spring 150.

As can be seen in Fig. 8, the second bearing 136 is pressed into the intermediate flange 130 by means of a first tool 400, in particular, a press tool.

While pressing the second bearing 136 into the intermediate flange 130, the intermediate flange 130 is countered into the pressing direction by a second tool 402, in particular, a press tool.

In other words, the second bearing 136 is press-fitted (e.g., press-connected or press-fixed) to the intermediate flange 130.

The leaf spring 150 is arranged on an opposite side of the intermediate flange 130 with respect to the side, in which the second bearing 136 is pressed.

Turning to Fig. 9, a part of the assembly method of Fig. 6 is schematically shown.

In particular, with reference to step S30, the first housing 126 being assembled with the electromagnet 108 of the brake device 100 and with the first bearing 134 is schematically shown.

In this respect, the electromagnet 108 is disposed in the first housing 126 and is fixed therein by means of at least one fastener 304, for example, two fasteners 304.

In other words, the electromagnet 108 is fixed to the first housing 126 by at least one fastener 304.

The fastener 304 is configured as a screw fastener.

A spacer 306 is arranged in the electromagnet 108, in particular in a passage for receiving the motor shaft 122, which is formed within the electromagnet 108.

The spacer 306 is pot shaped, i.e., the spacer 306 has a cylindrical hollow body with a collar at one end thereof, which collar protrudes outwards thereof.

The spacer 306 may be configured as a slide bearing.

As can be seen in Fig. 9, the first bearing 134 is pressed into the first housing 126 by means of a third tool 404, in particular, a press tool.

While pressing the first bearing 134 into the first housing 126, the first housing 126 is countered into the pressing direction by a fourth tool 406, in particular, a press tool.

In other words, the first bearing 134 is press-fitted (e.g., press-connected or press-fixed) to the first housing 126.

The first bearing 126 and the electromagnet 108 are disposed on the first housing 126 such as to sandwich the collar of the spacer 306 between themselves.

In other words, the collar of the spacer 306 is disposed between the first bearing 126 and the electromagnet 108, to thereby being fixed between them and, accordingly, to the first housing 126.

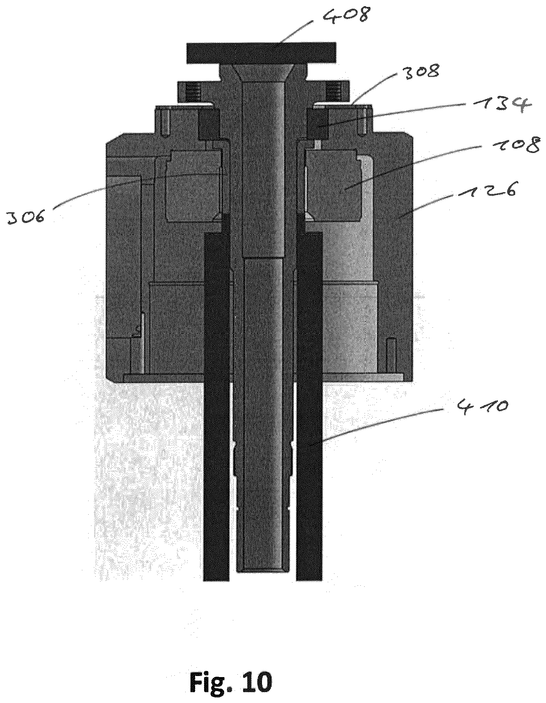

After pressing the first bearing 134 into the first housing 126, a plate 308 is fixed to the first housing 126 for reliably securing the seat of the first bearing 134 in the first housing 126 (see also Figs. 10 to 14).

In this respect, the plate 308 is disposed on the first housing 126 such as the first bearing 134 being sandwiched between the plate 308 and the first housing 126. For example, the plate 308 is disposed partly on top of the first bearing 134.

The plate 308 is fixed to the first housing 126 by means of at least one fastener, for example, two fasteners (not shown in Fig. 9, but see Figs. 10 to 14).

Said fastener is configured as a screw fastener.

Turning to Fig. 10, a part of the assembly method of Fig. 6 is schematically shown.

In particular, with reference to step S30, the first housing 126 being assembled with the motor shaft 122 is schematically shown. Especially, the first housing 126 being partly assembled as seen in Fig. 9 is further assembled with the motor shaft 122.

In this context, the expression partly assembled first housing 126 may be understood that the first housing 126 may be assembled or provided or mounted with a part of a/the plurality of elements of the motor stack 110 as particularly described with respect to Fig. 2A.

As can be seen in Fig. 10, the motor shaft 122 is pressed into the (partly assembled) first housing 126 by means of a fifth tool 408, in particular, a press tool.

Particularly, the motor shaft 122 is pressed into the first bearing 134 (i.e., the rotatable part of the first bearing 134) by means of the fifth tool 408 and extends through the spacer 306.

While pressing the motor shaft 122 into the (partly assembled) first housing 126, i.e., the first bearing 134 and through the spacer 306, the (partly assembled) first housing 126, in particular, the spacer 306 of the (partly assembled) first housing 126, is countered into the pressing direction by a sixth tool 410, in particular, a press tool.

In other words, the motor shaft 122 is press-fitted (e.g., press-connected or press-fixed) to the first bearing 134 (i.e., the rotatable part of the first bearing 134).

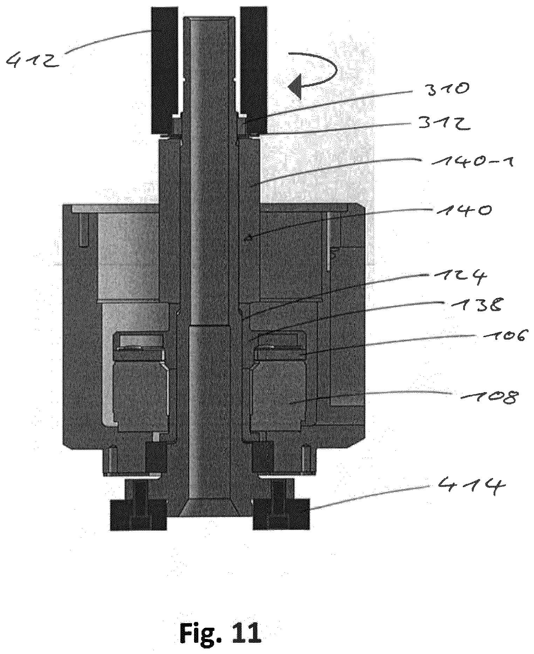

Turning to **Fig. 11****,** a part of the assembly method of Fig. 6 is schematically shown.

In particular, with reference to step S30, the first housing 126 being assembled with the motor shaft 122, the intermediate flange sub assembly, and the rotatable part 140-1 of the driving assembly 140 is schematically shown.

As can be seen in Fig. 11, the flange 138 together with the permanent magnet 106 (i.e., the intermediate flange sub assembly) is arranged on the motor shaft 122.

In particular, the flange 138 together with the permanent magnet 106 is disposed adjacent (e.g., next) to the spacer 306 and, accordingly, adjacent to the electromagnet 108.

On the motor shaft 122, the flange 138 is supported by the spacer 306.

In particular, the flange 138 is fixed to the motor shaft 122 by a/the above-described shaft hub connection 124.

In this regard, assembling / building tolerances, e.g., very defined assembling / building tolerances, may be important in order to secure the above-mentioned gap G between the electromagnet 108 and the permanent magnet 106 for obtaining the hysteresis effect.

Further, the rotatable part 140-1 of the driving assembly 140 is arranged on the motor shaft 122.

In particular, the rotatable part 140-1 of the driving assembly 140 is disposed adjacent (e.g., next) to the flange 138.

On the motor shaft 122, the rotatable part 140-1 of the driving assembly 140 is supported by the flange 138.

Hence, on the motor shaft 122, the flange 138 is disposed between the spacer 306 and the rotatable part 140-1 of the driving assembly 140.

Adjacent (e.g., next) to the rotatable part 140-1 of the driving assembly 140, fixing means for fixing the above-described elements (i.e., the flange 138 and the rotatable part 140-1 of the driving assembly 140) on the motor shaft 122 are disposed.

Said fixing means are configured as a slotted nut 310 and a retaining washer 312.

The retaining washer 312 is disposed adjacent (e.g., next) to the rotatable part 140-1 of the driving assembly 140.

Hence, on the motor shaft 122, the rotatable part 140-1 of the driving assembly 140 is disposed between the retaining washer 312 and the flange 138.

The slotted nut 310 is disposed adjacent (e.g., next) to the retaining washer 312.

In this respect, in a mounted state, the retaining washer 312 engages with the slotted nut 310 for fixing the slotted nut 310 in its mounted state on the motor shaft 122.

In other words, the retaining washer 312 is configured to engage a/the slotted nut 310 for fixing the slotted nut 310, e.g., for fixing the slotted nut 310 with respect to rotation.

The slotted nut 310 is screwed to a threaded portion of the motor shaft 122 by means of a seventh tool, in particular, a tool having an adapter for engaging a/the slotted nut 310, particularly, a torque wrench tool being configured for engaging a/the slotted nut 310.

Further, an eighth tool 414 is provided for mounting the slotted nut 310 to the motor shaft 122.

The motor shaft 122 and, hence, the (partly assembled) first housing 126 are temporarily mounted to the eighth tool 414, e.g., by means of fasteners, in particular, screw fasteners (for example two screw fasteners) for providing a fixation of the motor shaft 122 and, hence, the (partly assembled) first housing 126 during mounting the slotted nut 310.

In other words, the motor shaft 122 is temporarily fixed to the eighth tool 414 with respect to rotation during (e.g., rotatably) assembling the slotted nut 310.

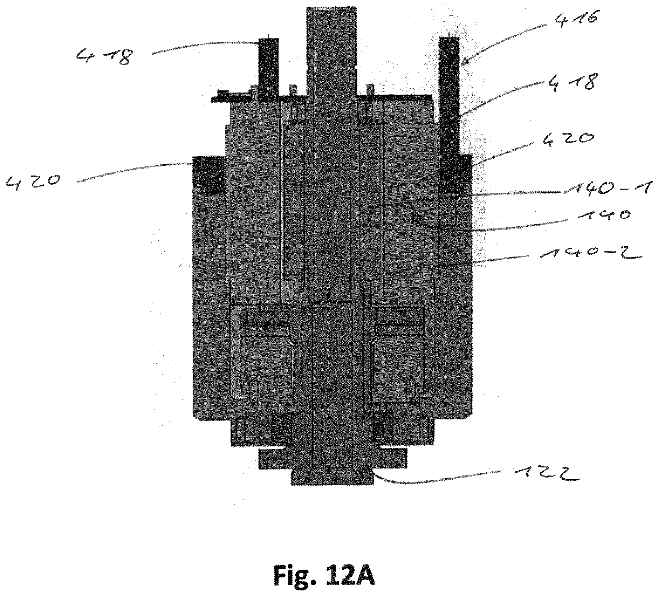

Turning to **Figs. 12A to 12C****,** alternatives of a part of the assembly method of Fig. 6 are schematically shown, respectively.

In particular, Figs. 12A to 12C respectively show different approaches of mounting the non-rotatable part 140-2 of the driving assembly 140 to the (partly assembled) first housing 126.

As can be seen in Figs. 12A to 12C, the non-rotatable part 140-2 of the driving assembly 140 is arranged in the (partly assembled) first housing 126.

Particularly, the non-rotatable part 140-2 of the driving assembly 140 is correspondingly disposed with respect to the rotatable part 140-1 of the driving assembly 140.

In this respect, the non-rotatable part 140-2 of the driving assembly 140 substantially surrounds the rotatable part 140-1 of the driving assembly 140.

The non-rotatable part 140-2 of the driving assembly 140 is fixed to the first housing 126 as known in the art, for example, by means of a form fit, or press fit, etc.

Now turning to **Fig. 12A****,** a first approach of said approaches is schematically shown.

A guide tool 416 for mounting the non-rotatable part 140-2 of the driving assembly 140 to the first housing 126 is provided.

The guide tool 416 comprises at least one guide rod 418 and at least one guide magnet 420.

For example, the guide tool 416 comprises three guide rods 418 and three guide magnets 420.

The guide rods 418 are evenly disposed around a mounting space for the first housing 126, i.e., the guide rods 418 are disposed around a/the mounting space for the first housing 126 at 120 degrees with respect to each other.

Each guide magnet 420 is assigned to a respective guide rod 418.

The respective guide magnet 420 is slidingly attached to its assigned guide rod 418.

Each guide magnet 420 is configured to attract the non-rotatable part 140-2 of the driving assembly 140 and to thereby hold the non-rotatable part 140-2 of the driving assembly 140 for mounting.

Hence, the (partly assembled) first housing 126 is disposed on the mounting space and the non-rotatable part 140-2 of the driving assembly 140 is manually clamped to the guide magnets 420 and mounted to the (partly assembled) first housing 126 in a sliding movement along said guide rods 418.

Then, in its final mounting state, the non-rotatable part 140-2 of the driving assembly 140 is manually released from the guide tool 416.

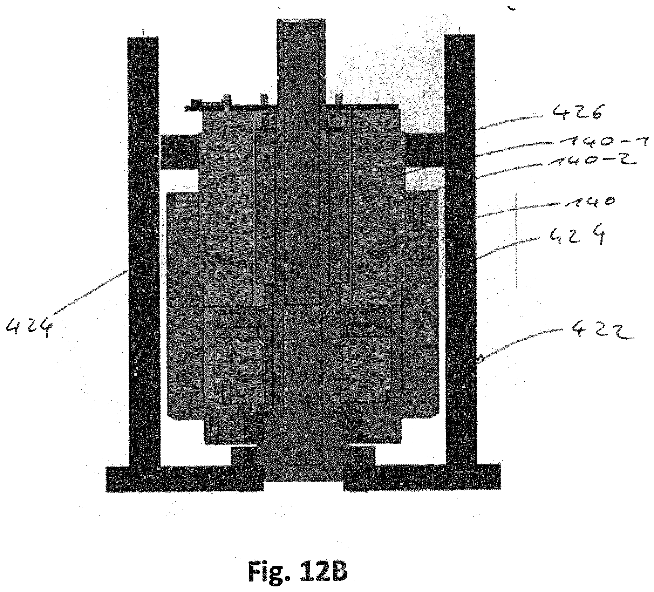

Now turning to **Fig. 12B****,** a second approach of said approaches is schematically shown.

A guide tool 422 for mounting the non-rotatable part 140-2 of the driving assembly 140 to the first housing 126 is provided.

The guide tool 422 comprises at least one guide rod 424 and at least one clamping device 426.

For example, the guide tool 422 comprises two guide rods 424 and one clamping device 426.

The guide rods 424 are disposed on opposite sides of a mounting space for the first housing 126, i.e., the guide rods 424 are disposed around a/the mounting space for the first housing 126 at 180 degrees with respect to each other and the mounting space.

The clamping device 426 is configured to surround the non-rotatable part 140-2 of the driving assembly 140 and to be reversibly arrangeable between a clamping state, in which the non-rotatable part 140-2 is clampable, and a releasing state, in which the non-rotatable part 140-2 is releasable.

For example, the clamping device 426 may be configured as a clamping ring or a clamping collar.

The clamping device 426 is fixed to the guide rods 424 and is able to move along the guide rods 424.

For mounting, the (partly assembled) first housing 126 is disposed on the mounting space and fixed to the guide tool 422 by means of fasteners, e.g., screw fasteners.

The non-rotatable part 140-2 of the driving assembly 140 is clamped in the clamping device 426 and (subsequently) mounted to the (partly assembled) first housing 126 in a sliding movement along said guide rods 424.

Then, in its final mounting state, the non-rotatable part 140-2 of the driving assembly 140 is released from the guide tool 422.

In the present embodiment, the clamping device 426 may be guided between the guide rods 424 (e.g., by means of a form fit) and/or may be guided over slide bushings and/or sleeve bearings arranged in the clamping device 426 for sliding along the guide rods 424.

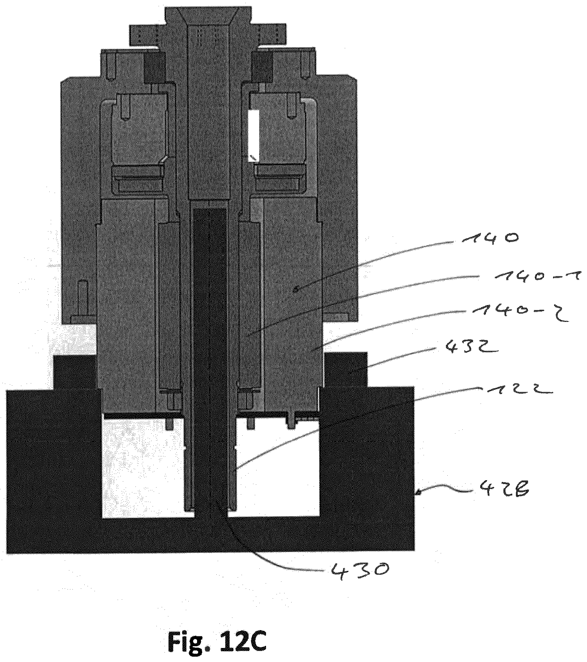

Now turning to **Fig. 12C****,** a third approach of said approaches is schematically shown.

A guide tool 428 for mounting the first housing 126 to the non-rotatable part 140-2 of the driving assembly 140 is provided.

The guide tool 428 comprises at least one guide rod 430 and at least one clamping device 432.

For example, the guide tool 422 comprises one guide rods 430 and one clamping device 432.

The guide rod 430 is disposed in a center of a mounting space for the non-rotatable part 140-2 of the driving assembly 140.

The clamping device 432 is configured to surround the non-rotatable part 140-2 of the driving assembly 140 and to be reversibly arrangeable between a clamping state, in which the non-rotatable part 140-2 is clampable, and a releasing state, in which the non-rotatable part 140-2 is releasable.

For example, the clamping device 432 may be configured as a clamping ring or a clamping collar.

For mounting, the non-rotatable part 140-2 is disposed on the mounting space and fixed thereto by means of the clamping device 432.

Then, the (partly assembled) first housing 126 is engaged to the guide rod 430 by means of the motor shaft 122, which is configured as a hollow shaft, by receiving the guide rod 430 in the motor shaft 122.

Subsequently, the (partly assembled) first housing 126 is assembled to the non-rotatable part 140-2 in a sliding movement along said guide rod 430.

Then, in its final mounting state, the (partly assembled) first housing 126 together with the non-rotatable part 140-2 of the driving assembly 140 is released from the guide tool 428.

Alternatively, it may also conceivable that the guide tool 428 comprises at least one guide rod being disposed on a side of the mounting place and at least one clamping device for clamping the (partly assembled) first housing 126 in a surrounding manner, wherein the clamping device is movable along the guide rod for mounting the (partly assembled) first housing 126 to the non-rotatable part 140-2 of the driving assembly 140.

Turning to **Fig. 13****,** a part of the assembly method of Fig. 6 is schematically shown.

In particular, with reference to step S40, the second housing 128 is mounted to the first housing 126, i.e., to the first housing sub assembly.

For example, the second housing 128 is screwed to the first housing 126, e.g., by means of a screw fastener.

Turning to **Fig. 14****,** a part of the assembly method of Fig. 6 is schematically shown.

In particular, with reference to step S50, the intermediate flange sub assembly is mounted to the second housing 128 and to the first housing sub assembly having the second housing 128 mounted thereto.

As can be seen in Fig. 14, the intermediate flange sub assembly is pressed onto the motor shaft 122 by means of a ninth tool 434, in particular, a press tool.

In particular, the second bearing 136, i.e., the rotatable part of the second bearing 136, is pressed onto the motor shaft 122 by means of a ninth tool 434, in particular, a press tool.

While pressing the second bearing 136 onto the motor shaft 122, the motor shaft 122 is countered into the pressing direction by a tenth tool 436, in particular, a press tool.

In other words, the second bearing 136 is press-fitted (e.g., press-connected or press-fixed) to the motor shaft 122.

### REFERENCES

- 10: joint arrangement
- 20: robot arm
- 100: brake device
- 102: stator assembly of brake device
- 104: rotor assembly of brake device
- 106: permanent magnet
- 108: electromagnet
- 110: motor stack
- 112: pulley
- 114: belt
- 116: control unit
- 118: rotor assembly of motor stack
- 120: stator assembly of motor stack
- 122: central motor shaft
- 124: shaft hub connection
- 126: first housing
- 128: second housing
- 130: intermediate flange
- 132: cover
- 134: first bearing
- 136: second bearing
- 138: flange
- 140: driving assembly
- 142: drive PCB
- 144: PCB holding structure
- 146: encoder
- 148: pretension spring
- 150: leaf spring
- 200: sub arm
- 202: further sub arm
- 204: adapter assembly
- 206: surgical instrument
- 300: brake armature
- 302: fastener
- 304: fastener
- 306: spacer
- 308: plate
- 310: slotted nut
- 312: retaining washer
- 400: first tool
- 402: second tool
- 404: third tool
- 406: fourth tool
- 408: fifth tool
- 410: sixth tool
- 412: seventh tool
- 414: eighth tool
- 416: guide tool
- 418: guide rod
- 420: guide magnet
- 422: guide tool
- 424: guide rod
- 426: clamping device
- 428: guide tool
- 430: guide rod
- 432: clamping device
- 434: ninth tool
- 436: tenth tool

- S1 to S4: steps of method
- S10 to S60: steps of assembly method

- G: gap

## Claims

1. Joint arrangement (10) for a robot arm (20), especially a robot arm (20) for use in microsurgery, comprising:
at least one brake device (100) being configured for engagement and for disengagement and comprising a stator assembly (102) and a rotor assembly (104), wherein the brake device (100) is further configured to be operable at a minimal power consumption in a disengagement state,
wherein the at least one brake device (100) further comprises:
at least one permanent magnet (106) for providing a braking effect during engagement and being assigned to the rotor assembly (104), and
at least one electromagnet (108) for releasing the braking effect during disengagement and being assigned to the stator assembly (102),
wherein the permanent magnet (106) and the electromagnet (108) are arranged or arrangeable to each other with a gap (G) between themselves to maintain a hysteresis effect between themselves to allow the brake device (100) to be operable at a minimal power consumption.

2. The joint arrangement (10) of claim 1,
wherein the gap (G) is smaller than 1 mm ± 10%, in particular smaller than 1 mm ± 5%. particularly smaller than 1 mm ± 3%, optionally, wherein the gap (G) is 0,5 mm ± 10%, in particular 0,5 mm ± 5%, particularly 0,5 mm ± 3%.

3. The joint arrangement (10) of one of claims 1 or 2,
wherein the joint arrangement (10) further comprises at least one motor stack (110) having a rotor assembly (118) and a stator assembly (120), wherein the brake device (100) is arranged in connection with or within the motor stack (110).

4. The joint arrangement (10) of claim 3,
wherein the rotor assembly (104) of the brake device (100) is configured as a part of the rotor assembly (118) of the motor stack (110), and the stator assembly (102) of the brake device (100) is configured as a part of the stator assembly (120) of the motor stack (110).

5. The joint arrangement (10) of claim 3 or 4,
wherein the motor stack (110) comprises at least one spring (150), in particular at least one leaf spring (150), being configured to provide a spring force to the brake device (100).

6. Robot arm (20), especially robot arm (20) for use in microsurgery, comprising:
a joint arrangement (10) of one of claims 1 to 5.

7. Method for operating a brake device (100) in a joint arrangement (10) for a robot arm (20), especially a robot arm (20) for use in microsurgery, the method at least comprising the following step:
the brake device (100) is provided and configured for engagement and for disengagement, the brake device (100) comprising a stator assembly (102) and a rotor assembly (104),
wherein the brake device (100) is further operated at a minimal power consumption in a disengagement state,
wherein the brake device (100) further comprises:
at least one permanent magnet (106) for providing a braking effect during engagement and being assigned to the rotor assembly (104), and
at least one electromagnet (108) for releasing the braking effect during disengagement and being assigned to the stator assembly (102),
wherein the permanent magnet (106) and the electromagnet (108) are arranged or arrangeable to each other with a gap (G) between themselves to maintain a hysteresis effect between themselves to allow the brake device (100) to be operable at a minimal power consumption.

8. The method of claim 7,
wherein the brake device (100) is further configured for engagement at an engagement threshold value and for disengagement at a disengagement threshold value being higher than the engagement threshold value, and
the method further comprises the following steps:
providing full power to the brake device (100) by applying Pulse Width Modulation (PWM) for disengaging the brake device (100),
reducing the power provided to the brake device (100) to a first predetermined power value by applying PWM, wherein the first predetermined power value is set between the disengagement threshold value and the engagement threshold value, and
maintaining the power provided to the brake device (100) at the first predetermined value by applying PWM for keeping the brake device (100) in disengagement.

9. The method of claim 8,
wherein the method further comprises reducing the power provided to the brake device (100) to a second predetermined power value by applying PWM for engaging the brake device (100), wherein the second predetermined power value is set below the engagement threshold value.

10. The method of claims 8 or 9,
wherein the first predetermined power value is half of the full power being providable or provided to the brake device (100) by applying PWM for disengaging the brake device (100).

11. The method of one of claims 8 to 10,
wherein the full power being providable or provided to the brake device (100) by applying PWM for disengaging the brake device (100) includes a 24V signal.

12. The method of one of claims 8 to 11,
wherein the disengagement threshold value is 5/8 of the full power.

13. The method of one of claims 8 to 12,
wherein the engagement threshold value is 1/3 of the full power.

14. The method of one of claims 8 to 13,
wherein providing full power to the brake device (100) by applying PWM for disengaging the brake device (100) is maintained for a first predetermined time period, and maintaining the power provided to the brake device (100) at the first predetermined power value by applying PWM for keeping the brake device (100) in disengagement is maintained for a second predetermined time period,
wherein the second predetermined time period is longer than the first predetermined time period.

15. The method of one of claims 7 to 14,
wherein the brake device (100) comprises at least one permanent magnet (106) for providing a braking effect during engagement and at least one electromagnet (108) for releasing the braking effect during disengagement.

16. The method of one of claims 7 to 15,
wherein the brake device (100) is arranged in connection with or within a motor stack (110) of the joint arrangement (10), optionally, wherein the motor stack (110) comprises at least one spring (150), in particular at least one leaf spring (150), being configured to provide a spring force to the brake device (100).

## Patentansprüche

1. Gelenkanordnung (10) für einen Roboterarm (20), insbesondere einen Roboterarm (20) zur Verwendung in der Mikrochirurgie, umfassend:
mindestens eine Bremsvorrichtung (100), die zur Einkopplung und zur Auskopplung ausgelegt ist und eine Statorbaugruppe (102) sowie eine Rotorbaugruppe (104) umfasst,
wobei die Bremsvorrichtung (100) ferner so ausgelegt ist, dass sie in einem ausgekoppelten Zustand mit minimaler Leistungsaufnahme betrieben werden kann,
wobei die mindestens eine Bremsvorrichtung (100) ferner umfasst:
mindestens einen Permanentmagneten (106) zur Erzeugung einer Bremswirkung während des eingekoppelten Zustands, der der Rotorbaugruppe (104) zugeordnet ist, und
mindestens einen Elektromagneten (108) zum Aufheben der Bremswirkung während des ausgekoppelten Zustand, der der Statorbaugruppe (102) zugeordnet ist,
wobei der Permanentmagnet (106) und der Elektromagnet (108) mit einem Spalt (G) zueinander angeordnet sind oder anordenbar sind, um einen Hystereseeffekt zwischen sich aufrechtzuerhalten, damit die Bremsvorrichtung (100) mit minimalem Stromverbrauch betrieben werden kann.

2. Die Gelenkanordnung (10) nach Anspruch 1,
wobei der Spalt (G) kleiner als 1 mm ± 10 %, insbesondere kleiner als 1 mm ± 5 %, besonders kleiner als 1 mm ± 3 % ist, wobei der Spalt (G) optional 0,5 mm ± 10 %, insbesondere 0,5 mm ± 5 %, besonders 0,5 mm ± 3 % beträgt.

3. Die Gelenkanordnung (10) nach einem der Ansprüche 1 oder 2,
wobei die Gelenkanordnung (10) ferner mindestens einen Motorstapel (110) mit einer Rotorbaugruppe (118) und einer Statorbaugruppe (120) umfasst, wobei die Bremsvorrichtung (100) in Verbindung mit oder innerhalb des Motorstapels (110) angeordnet ist.

4. Die Gelenkanordnung (10) nach Anspruch 3,
wobei die Rotorbaugruppe (104) der Bremsvorrichtung (100) als Teil der Rotorbaugruppe (118) des Motorstapels (110) ausgebildet ist und die Statorbaugruppe (102) der Bremsvorrichtung (100) als Teil der Statorbaugruppe (120) des Motorstapels (110) ausgebildet ist.

5. Die Gelenkanordnung (10) nach Anspruch 3 oder 4,
wobei der Motorstapel (110) mindestens eine Feder (150), insbesondere mindestens eine Blattfeder (150), umfasst, die so ausgebildet ist, dass sie der Bremsvorrichtung (100) eine Federkraft zuführt.

6. Roboterarm (20), insbesondere Roboterarm (20) zur Verwendung in der Mikrochirurgie, umfassend: eine Gelenkanordnung (10) nach einem der Ansprüche 1 bis 5.

7. Verfahren zum Betreiben einer Bremsvorrichtung (100) in einer Gelenkanordnung (10) für einen Roboterarm (20), insbesondere einen Roboterarm (20) zur Verwendung in der Mikrochirurgie, wobei das Verfahren mindestens den folgenden Schritt umfasst:
die Bremsvorrichtung (100) ist zur Einkopplung und zur Auskopplung vorgesehen und ausgebildet, wobei die Bremsvorrichtung (100) eine Statorbaugruppe (102) und eine Rotorbaugruppe (104) umfasst,
wobei die Bremsvorrichtung (100) in einem ausgekoppeltenZustand ferner mit minimaler Leistungsaufnahme betrieben wird,
wobei die Bremsvorrichtung (100) ferner umfasst:
mindestens einen Permanentmagneten (106) zur Erzeugung einer Bremswirkung während des eingekoppelten Zustands, der der Rotorbaugruppe (104) zugeordnet ist, und
mindestens einen Elektromagneten (108) zum Aufheben der Bremswirkung während des ausgekoppelten Zustands, der der Statorbaugruppe (102) zugeordnet ist,
wobei der Permanentmagnet (106) und der Elektromagnet (108) mit einem Spalt (G) zueinander angeordnet sind oder anordenbar sind, um einen Hystereseeffekt zwischen sich aufrechtzuerhalten, damit die Bremsvorrichtung (100) bei minimalem Stromverbrauch betrieben werden kann.

8. Verfahren nach Anspruch 7,
wobei die Bremsvorrichtung (100) ferner so konfiguriert ist, dass sie bei einem Einkopplungs-Schwellenwert einkoppelt und bei einem Auskopplungs-Schwellenwert auskoppelt, wobei der Auskopplungs-Schwellenwert höher ist als der Einkopplungs-Schwellenwert, und
das Verfahren ferner die folgenden Schritte umfasst:
Bereitstellung der vollen Leistung für die Bremsvorrichtung (100) durch Anwendung einer Pulsweitenmodulation (PWM) zum auskoppeln der Bremsvorrichtung (100),
Reduzieren der der Bremsvorrichtung (100) zugeführten Leistung auf einen ersten vorgegebenen Leistungswert durch Anwendung von PWM, wobei der erste vorgegebene Leistungswert zwischen dem Auskopplungs-Schwellenwert und dem Einkopplungs-Schwellenwert festgelegt ist, und
Aufrechterhalten der der Bremsvorrichtung (100) zugeführten Leistung auf dem ersten vorgegebenen Wert durch Anwendung von PWM, um die Bremsvorrichtung (100) im ausgekoppelten Zustand zu halten.

9. Verfahren nach Anspruch 8,
wobei das Verfahren ferner umfasst: Verringern der der Bremsvorrichtung (100) zugeführten Leistung auf einen zweiten vorgegebenen Leistungswert durch Anwenden von PWM, um die Bremsvorrichtung (100) einzukoppeln, wobei der zweite vorgegebene Leistungswert unterhalb des Einkopplungs-Schwellenwerts festgelegt ist.

10. Verfahren nach Anspruch 8 oder 9,
wobei der erste vorgegebene Leistungswert die Hälfte der vollen Leistung beträgt, die der Bremsvorrichtung (100) durch Anwendung von PWM zurAuskopplungder Bremsvorrichtung (100) zugeführt werden kann oder zugeführt wird.

11. Verfahren nach einem der Ansprüche 8 bis 10,
wobei die volle Leistung, die der Bremsvorrichtung (100) durch Anwendung von PWM zum Auskoppeln der Bremsvorrichtung (100) bereitgestellt werden kann oder bereitgestellt wird, ein 24-V-Signal umfasst.

12. Verfahren nach einem der Ansprüche 8 bis 11,
wobei der Auskopplungs-Schwellenwert 5/8 der vollen Leistung beträgt.

13. Verfahren nach einem der Ansprüche 8 bis 12,
wobei der Einkopplungs-Schwellenwert 1/3 der vollen Leistung beträgt.

14. Verfahren nach einem der Ansprüche 8 bis 13,
wobei die Bereitstellung der vollen Leistung an die Bremsvorrichtung (100) durch Anwendung von PWM zum Auskopplung der Bremsvorrichtung (100) für einen ersten vorbestimmten Zeitraum aufrechterhalten wird und
das Aufrechterhalten der der Bremsvorrichtung (100) zugeführten Leistung auf dem ersten vorgegebenen Leistungswert durch Anwendung von PWM, um die Bremsvorrichtung (100) im ausgekoppelten Zustand zu halten, über einen zweiten vorgegebenen Zeitraum aufrechterhalten wird,
wobei der zweite vorbestimmte Zeitraum länger ist als der erste vorbestimmte Zeitraum.

15. Verfahren nach einem der Ansprüche 7 bis 14,
wobei die Bremsvorrichtung (100) mindestens einen Permanentmagneten (106) zum Erzeugen einer Bremswirkung während des eingekoppelten Zustands und mindestens einen Elektromagneten (108) zum Aufheben der Bremswirkung während des ausgekoppelten Zustands umfasst.

16. Verfahren nach einem der Ansprüche 7 bis 15,
wobei die Bremsvorrichtung (100) in Verbindung mit oder innerhalb eines Motorstapels (110) der Gelenkanordnung (10) angeordnet ist, wobei der Motorstapel (110) gegebenenfalls mindestens eine Feder (150), insbesondere mindestens eine Blattfeder (150), umfasst, die so ausgelegt ist, dass sie eine Federkraft auf die Bremsvorrichtung (100) ausübt.

## Revendications

1. Dispositif d'articulation (10) pour un bras de robot (20), en particulier un bras de robot (20) destiné à être utilisé en microchirurgie, comprenant :
au moins un dispositif de freinage (100) configuré pour s'engager et se désengager et comprenant un ensemble stator (102) et un ensemble rotor (104),
dans lequel le dispositif de freinage (100) est en outre conçu pour fonctionner avec une consommation d'énergie minimale dans un état de désengagement,
dans lequel ledit au moins un dispositif de freinage (100) comprend en outre :
au moins un aimant permanent (106) destiné à fournir un effet de freinage pendant l'engagement et associé à l'ensemble rotor (104), et
au moins un électroaimant (108) destiné à supprimer l'effet de freinage pendant le désengagement et associé à l'ensemble stator (102),
dans lequel l'aimant permanent (106) et l'électroaimant (108) sont agencés ou peuvent être agencés l'un par rapport à l'autre avec un entrefer (G) entre eux afin de maintenir un effet d'hystérésis entre eux pour permettre au dispositif de freinage (100) de fonctionner avec une consommation d'énergie minimale.

2. Dispositif d'articulation (10) selon la revendication 1,
dans lequel l'entrefer (G) est inférieur à 1 mm ± 10 %, en particulier inférieur à 1 mm ± 5 %, plus particulièrement inférieur à 1 mm ± 3 %, et, le cas échéant, dans lequel l'entrefer (G) est de 0,5 mm ± 10 %, en particulier de 0,5 mm ± 5 %, plus particulièrement de 0,5 mm ± 3 %.

3. Dispositif d'articulation (10) selon l'une des revendications 1 ou 2,
dans lequel l'ensemble de jonction (10) comprend en outre au moins un bloc moteur (110) comportant un ensemble rotor (118) et un ensemble stator (120), le dispositif de freinage (100) étant agencé en liaison avec ou à l'intérieur du bloc moteur (110).

4. Dispositif d'articulation (10) selon la revendication 3,
dans lequel l'ensemble rotor (104) du dispositif de freinage (100) est configuré comme une partie de l'ensemble rotor (118) du bloc moteur (110), et l'ensemble stator (102) du dispositif de freinage (100) est configuré comme une partie de l'ensemble stator (120) du bloc moteur (110).

5. Dispositif d'articulation (10) selon la revendication 3 ou 4,
dans lequel le bloc-moteur (110) comprend au moins un ressort (150), en particulier au moins un ressort à lame (150), configuré pour fournir une force élastique au dispositif de freinage (100).

6. Bras robotisé (20), en particulier bras robotisé (20) destiné à être utilisé en microchirurgie, comprenant : un agencement d'articulation (10) selon l'une des revendications 1 à 5.

7. Procédé de commande d'un dispositif de freinage (100) dans un dispositif d'articulation (10) pour un bras robotisé (20), en particulier un bras robotisé (20) destiné à être utilisé en microchirurgie, le procédé comprenant au moins l'étape suivante :
le dispositif de freinage (100) est prévu et configuré pour être enclenché et désenclenché, le dispositif de freinage (100) comprenant un ensemble stator (102) et un ensemble rotor (104),
dans lequel le dispositif de freinage (100) est en outre actionné avec une consommation d'énergie minimale dans un état de désengagement,
dans lequel le dispositif de freinage (100) comprend en outre :
au moins un aimant permanent (106) destiné à fournir un effet de freinage pendant l'engagement et associé à l'ensemble rotor (104), et
au moins un électroaimant (108) destiné à supprimer l'effet de freinage lors du désengagement et associé à l'ensemble stator (102),
dans lequel l'aimant permanent (106) et l'électroaimant (108) sont agencés ou peuvent être agencés l'un par rapport à l'autre avec un entrefer (G) entre eux afin de maintenir un effet d'hystérésis entre eux pour permettre au dispositif de freinage (100) de fonctionner avec une consommation d'énergie minimale.

8. Procédé selon la revendication 7,
dans lequel le dispositif de freinage (100) est en outre configuré pour s'enclencher à une valeur seuil d'enclenchement et pour se désenclencher à une valeur seuil de désenclenchement supérieure à la valeur seuil d'enclenchement, et
le procédé comprend en outre les étapes suivantes :
fournir la pleine puissance au dispositif de freinage (100) en appliquant une modulation de largeur d'impulsion (PWM) pour désengager le dispositif de freinage (100),
réduire la puissance fournie au dispositif de freinage (100) à une première valeur de puissance prédéterminée en appliquant la modulation PWM, la première valeur de puissance prédéterminée étant fixée entre la valeur seuil de désengagement et la valeur seuil d'engagement, et
maintenir la puissance fournie au dispositif de freinage (100) à la première valeur prédéterminée en appliquant la modulation PWM afin de maintenir le dispositif de freinage (100) en désengagement.

9. Procédé selon la revendication 8,
dans lequel le procédé comprend en outre la réduction de la puissance fournie au dispositif de freinage (100) à une deuxième valeur de puissance prédéterminée en appliquant une modulation d'impulsions en largeur (PWM) pour engager le dispositif de freinage (100), la deuxième valeur de puissance prédéterminée étant fixée en dessous de la valeur seuil d'engagement.

10. Procédé selon la revendication 8 ou 9,
dans lequel la première valeur de puissance prédéterminée correspond à la moitié de la puissance maximale pouvant être fournie ou fournie au dispositif de freinage (100) en appliquant une modulation d'impulsions en largeur (PWM) pour désengager le dispositif de freinage (100).

11. Procédé selon l'une des revendications 8 à 10,
dans lequel la puissance maximale pouvant être fournie ou fournie au dispositif de freinage (100) en appliquant une modulation d'impulsions en largeur (PWM) pour désengager le dispositif de freinage (100) comprend un signal de 24 V.

12. Procédé selon l'une des revendications 8 à 11,
dans lequel la valeur seuil de désengagement est égale à 5/8 de la puissance maximale.

13. Procédé selon l'une des revendications 8 à 12,
dans lequel la valeur seuil d'engagement est égale à 1/3 de la puissance maximale.

14. Procédé selon l'une des revendications 8 à 13,
dans lequel la fourniture de la puissance maximale au dispositif de freinage (100) par application d'une modulation d'impulsions en largeur (PWM) pour désengager le dispositif de freinage (100) est maintenue pendant une première période de temps prédéterminée, et
le maintien de la puissance fournie au dispositif de freinage (100) à la première valeur de puissance prédéterminée par application d'une modulation d'impulsions en largeur (PWM) pour maintenir le dispositif de freinage (100) désengagé est maintenu pendant une deuxième période de temps prédéterminée,
dans lequel la deuxième durée prédéterminée est plus longue que la première durée prédéterminée.

15. Procédé selon l'une des revendications 7 à 14,
dans lequel le dispositif de freinage (100) comprend au moins un aimant permanent (106) destiné à produire un effet de freinage pendant l'engagement et au moins un électroaimant (108) destiné à supprimer l'effet de freinage pendant le désengagement.

16. Procédé selon l'une des revendications 7 à 15,
dans lequel le dispositif de freinage (100) est agencé en liaison avec ou à l'intérieur d'un ensemble de moteurs (110) du dispositif d'articulation (10), le cas échéant, dans lequel l'ensemble de moteurs (110) comprend au moins un ressort (150), en particulier au moins un ressort à lames (150), configuré pour exercer une force élastique sur le dispositif de freinage (100).
